# EUROPEAN PATENT APPLICATION

(11) **EP 3 373 011 A1**
(43) Date of publication of application: **12.09.2018**
(21) Application number: 16861716.5
(22) Date of filing: 06.11.2016
(51) Int. Cl.: G01N 33/577, G01N 33/551, G01N 21/78, G01N 21/31

(54) **METHIONINE ADENOSYLTRANSFERASE BIOLOGICAL ACTIVITY ASSAY METHOD AND REAGENT KIT**

(30) Priority: 07.11.2015 CN 201510755262
(71) Applicant: Hunan Skyworld Biotechnologies Co., Changsha, Hunan 410100 (CN)
(72) Inventor: HAO, Xiujuan, Changsha Hunan 410100 (CN); LI, Huijun, Changsha Hunan 410100 (CN); ZHOU, Min, Changsha Hunan 410100 (CN)
(74) Representative: dompatent von Kreisler Selting Werner - Partnerschaft von Patent- und Rechtsanwälten mbB
(86) International application number: PCT/IB2016/056666
(87) International publication number: WO 2017/077509

(57) **Abstract**

The present invention discloses a methionine adenosyltransferase (MAT) activity assay method and a kit for measuring MAT activity. A sample and relevant reagents are mixed in certain way that MAT-catalyzed reaction occurs efficiently. The reaction and the competitive ELISA that quantifies the product S-adenosylmethionine (SAM) are carried out simultaneously. The MAT activity is calculated as the amount of SAM produced per unit time. SAM is calculated through spectral absorbance of the SAM produced and comparing it to that of the standard. The method of SAM quantification is via tracer-labelled anti-SAM antibody or SAM (or SAM analog) antigen through competitive ELISA, so that the produced SAM competes with the SAM antigen for binding anti-SAM antibody. The method and the kit described in the present invention are more sensitive, accurate, reliable, straightforward, easier and faster. The method was used to measure the MAT activities of normal and cancerous liver cells.

## Description

This application is a 371 application of International Application No. PCT/IB2016/ 056666 filed November 6, 2016; (published on May 11, 2017 as WO/2017/077509) titled "Methionine Adenosyltransferase (MAT) Biological Activity Assay Method and Reagent Kit"; the entire contents of which are hereby incorporated by reference herewith. This application also claims priority to CN Application No. 201510755262.3 filed November 7, 2015.

### FIELD OF THE INVENTION

The present invention relates to the detection of biological samples, more in particular to a method and a kit for determining the biological activity of a methionine adenosyltransferase (MAT).

### BACKGROUND OF THE INVENTION

In addition to host-dependent parasites, Methionine Adenosyltransferase (MAT, EC2.5.1.6), also known as S-adenosylmethionine synthetase, is present in the cells of all species of organisms. The MAT sequence is very highly conserved, with a 59% homology in the gene sequence of MAT between human and *E. coli.* In mammals, MAT contains three isoenzymes encoded by three genes. MAT-I and MAT-III are composed of the catalytic subunit α1 encoded by the same gene (MAT1a). MAT-I is a tetramer and MAT-III is a dimer mainly present in adult liver cells. MAT-II is composed of catalytic subunit α2 encoded by another MAT gene (MAT2a) and is present in cells other than liver, embryonic liver and liver cancer cells. The MAT2 beta gene encodes a regulatory subunit beta that primarily regulates MAT-II. The catalyzed reaction of MAT *in vivo* is divided into two steps: (1) Catalyzing methionine (Met) and adenosine triphosphate (ATP) to produce S-adenosylmethionine (commonly known as activated or active methionine, S-adenosylmethionine, SAM, AdoMet) and tripolyphosphate (PPPi). SAM and PPPi remain on the MAT surface; and (2) the phosphatase activity of MAT will further decompose PPPi into diphosphoric acid (PPi) and inorganic monophosphoric acid (Pi).

MAT catalyzes the production of S-adenosylmethionine from L-methionine or methionine (L-Met) and adenosine triphosphate (ATP). MAT has a triphosphatase activity that degrades triphosphates into pyrophosphate and phosphoric acid. It's enzymatic activity is dependent on Mg²⁺ and K⁺, and the enzymatic reaction is as follows:

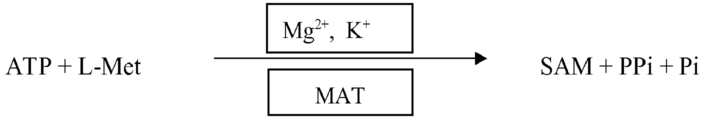

ATP and SAM are the most common metabolic intermediates in organisms. In the methionine cycle, methionine is first activated to form SAM. SAM provides methyl groups to important substances in life, such as DNA, RNA, lipoproteins, hormones, neurotransmitters, etc., and becomes homocysteine (HCY). Finally, through the methyl group provided by tetrahydrofolate, HCY becomes methionine. In the liver, the methionine cycle has an additional function, mainly, to quickly reduce the high level of methionine in the blood after a high methionine or high-protein diet, and finally through homocysteine, cysteine, cystathionine, and glutathione, the metabolites are transported to other organs.

SAM is the only sulphur-containing bioactive substance in nature with extremely diversified and important functions. It is the key substance in the methionine cycle and is the only methyl donor in the methylation modification process that is critical to humans. It plays an important role in transmethylation, trans-sulfuration and transamidation reactions. There are direct effects on methylation-related cellular functions, polyamine synthesis, and the ratio of methionine to homocysteine. It is of great importance in various life-related metabolic reactions and cell proliferation and differentiation. The SAM-dependent methyltransferase accounts for 1% of the total number of genes in the human genome. Studies have shown that maintaining a certain concentration of SAM in the liver plays a crucial role in the function of liver function. About 85% of methylation reactions and about 50% of methionine metabolism are performed in the liver, not only suggesting that the liver plays an important role in regulating blood methionine levels (by enhancing MAT-I/III activity), but also that SAM plays important role in the liver cell regeneration, differentiation and sensitivity to hepatocellular damage caused by various factors (alcohol, chemicals, radiation, pathogenic microorganisms, viruses, parasites, etc.). Therefore, it is possible to accurately, sensitively, and conveniently measure the biological activity of MAT and the content of SAM under different conditions, which provides an important insight for deepening the understanding of the methionine cycle and provides critical research tools for studying the regulations between different tissues and organs under different physiological and pathological conditions.

Partial inactivation of the MAT enzyme was observed in liver injury due to hepatic inflammation or oxidative stress. The MAT1a gene is not expressed at all in liver cirrhosis and liver cancer. The level of SAM decreased in MATI/III-deficient mice; in mice lacking glycine Nmethyltransferase (GNMT), the level of SAM increased. In both cases, the probability of liver cancer in mice increased significantly.

Many studies have shown that MAT and its catalytically synthesized SAMs play crucial roles in different stages of human life, such as embryogenesis, development, growth, differentiation, health, and disease, because SAM has very important special roles in the methionine cycle and carbon metabolism, and is directly related to the metabolism of the human body. The body's SAM content fluctuates based on age, gender, race, weight, diet, medication status, health, and illness. In view of this characteristic, both the synthesis and decomposition of SAM should be considered at the same time. Therefore, understanding the levels of biological activity of MAT under different situations has great practical significance for us to study many vital metabolic and health problems.

There are two configurations of the MAT enzyme active center, with the first configuration favoring SAM synthesis, and the second configuration having triphosphate hydrolysis activity. The enzyme configuration of the second configuration is significantly slower than the first configuration. The conversion from the first configuration to the second configuration takes some time. The difference between the two configurations lies in the sensitivity to the triphosphatase and nitrosylation.

There are currently two methods for determining MAT activity: In method 1, MAT eventually produces Pi under the conditions of the saturated substrates methionine, adenosine triphosphate and PPPi. The content of inorganic phosphorus Pi was measured by direct staining of malachite green and ammonium molybdate. In method 2, the amount of SAM catalyzed by MAT was determined by high pressure liquid chromatography (HPLC) or mass spectrometry (LC-MS/MS).
Among the above methods, method 1 has a big limitation: (1) PPPi, PPi and Pi coexist in the reaction system, and malachite green and ammonium molybdate also have some combination with PPPi and PPi, and the measured Pi value is inaccurate; (2) Sensitivity is not high; (3) MAT's triphosphatase activity requires configuration transformation, therefore, measurement of the reaction product Pi for the determination of the triphosphatase has a hysteresis effect. The indirect method measures the delayed product Pi, hence the variability of this method is high; (4) The triphosphatase activity of MAT is greatly affected by Met, which impacts the stability of the method; (5) The use of triphosphatase activity of MAT to represent the SAM synthesis ability (or activity) of MAT has a limitation or defect. It is very likely that the two enzyme activities of MAT are disproportionately related. It is highly possible that different MAT enzyme activities (referring to the ability of SAM synthesis and the ability of PPPi to hydrolyze) are differently influenced by different factors, making it difficult to use MAT's phosphatase hydrolysis activity to accurately reflect the SAM synthesis activity of MAT.

Fernández-Irigoyen reported that the MAT-I and MAT-III enzyme activities of different mutant strains were different from those of wild-type MAT-I and MAT-III in that their triphosphate hydrolase activity and SAM synthesis activity were affected differently. The activity of triphosphatase was unchanged, but the SAM synthesis activity was significantly reduced in some mutants; other mutant strains had enhanced SAM synthesis ability but triphosphoric acid hydrolase activity decreased; and yet some others had two types of enzyme activities that were both reduced to various degrees. In short, all kinds of possibilities exist.

Sanxhez del Pino et al. found that MAT-III introduces an NO group at 121 cysteine residue (nitrosation), which results in a marked decrease in SAM synthesis activity but no inhibition of triphosphatase activity of MAT. Therefore, determining MAT activity by measuring Pi can only reflect the overall results of SAM synthetase activity and triphosphatase activity, but cannot reflect its SAM synthesis ability. The result is inaccurate and therefore has great limitations.

In the Fernández-Irigoyen's report, measurement of SAM synthesis activity is based on the use of method 2 mentioned above, that is, HPLC method to measure the amount of SAM generated. In fact, no matter which method is used, either HPLC or LC-MS/MS to measure the concentration of SAM, and hence evaluate MAT activity, it is very inaccurate. We know that SAM is bound to MAT for a period of time after its synthesis, whereas HPLC and LC-MS/MS methods (1) only measure free SAM molecules; (2) samples used for HPLC and LC-MS/MS platforms have to undergo special treatment, after a long time and many steps, the content of SAM must reults in some loses; (3) the amount of SAM cannot be determined in time (SAM molecule itself is very unstable, therefore SAM needs to be measured timely without delay). All these factors will cause the measured SAM value rather inaccurate, let alone trying to achieve the purpose of reflecting the enzyme kinetic reaction.

Therefore, it is necessary to develop a detection method that can accurately, rapidly and directly determine the biological activity of methionine adenosyltransferase.

### SUMMARY OF THE INVENTION

The object of the present invention is to provide a method for accurately, rapidly and directly measuring the biological activity of methionine adenosyltransferase and a kit developed by the method. The one-step method or the simultaneous method to directly determine the concentration of the synthesized SAM product involved in the present invention is an innovation in the field of MAT activity measurement. MAT is the only enzyme synthesizing SAM. The present invention can measure the concentration of SAM most rapidly and directly for the first time to determine the level of MAT activity and SAM concentration at the same time ex *vivo* or *in vitro.* In the present invention, the MAT catalyzed biochemical reaction and the determination of the SAM immunoreaction are performed simultaneously and the two processes are seamlessly combined, which is particularly significant for accurately measuring the SAM molecule that is unstable by nature. The novelty of the present invention is the determination of the biological activity of methionine adenosyltransferase using anti-S-adenosylmethionine-specific antibodies. The method for determining the biological activity of the methionine adenosyltransferase comprises the following steps: (1) In a buffer system that ensures the biological activity of methionine adenosyltransferase, the sample containing the enzyme and the substrate react to produce certain amount of S-adenosylmethionine product; (2) The use of immunological methods to measure the level of S-adenosylmethionine, in order to determine whether the reaction system possesses methionine adenosyltransferase activity, as well as the level of its activity. Among them, the substrate contains methionine, adenosine triphosphate, magnesium ion and potassium ion in a suitable acid-alkaline buffer system; the sample can be derived from genetic engineering products, purified samples from cells of biological tissues, methionine adenosyltransferases in the tissue cells, biological fluids or tissue and cell cultured fluids, biological fluids including blood, plasma, serum, saliva, urine, cerebrospinal fluid, abdominal or thoracic exudates and tissue fluids. Immunoassays can be made up of anti- S-adenosylmethionine-specific antibody, various tracer-coupled conjugates to such antibody, S-adenosylmethionine or its analogues, and conjugated-S-adenosylmethionine or its conjugated analogues. Catalytic reaction of methionine adenosyltransferase and its resulting product S-adenosine methionine competes with the coated S-adenosylmethionine antigen or tracer-labeled S-adenosylmethionine antigen in binding tracer-labeled anti- S-adenosylmethionine antibody or coated anti-S-adenosine antibody. The immune response is based on the one-step principle that enzymatic reaction and immunoassay are performed simultaneously.

In order to solve the problems existing in the prior art (measurement of inorganic phosphorus Pi content by direct staining of malachite green and ammonium molybdate; HPLC or LC-MS/MS method), the present invention provides a method for determining the biological activity of methionine adenosyltransferase in a sample. One of the formats is as follows:
(1) Preparation of a standard with different concentrations: Take the S-adenosylmethionine standard in the kit and prepare it in the proper buffer at different concentrations used for a standard curve;
(2) In a microtiter plate coated with a protein (or polymer)-SAM antigen conjugates or protein (or polymer)-SAM analogue antigen conjugates, add the standards of different concentrations to the standard wells and samples to be tested to sample wells;
(3) Add horseradish peroxidase or alkaline phosphatase labeled anti-S-adenosylmethionine monoclonal antibody, methionine, adenosine triphosphate, and methionine adenosine transferase positive controls, and mix them for 60 min at 37°C. The plate is washed after the reaction;
(4) Add horseradish peroxidase or alkaline phosphatase substrate, allow color development at 37 °C for 15 min, stop solution was added to terminate the reaction, and read optical absorbance at 450nm wavelength per well;
(5) In the same enzyme-linked immunoassay plate, a series of SAM with known gradients are used as standards to plot a standard curve. The curve equation is obtained based on the values of OD450 and the known standard concentrations, and then the sample OD450 is substituted into the equation to obtain the corresponding SAM concentration;
(6) The MAT enzyme activity is calculated based on the concentration of catalyzed product SAM per unit time of a certain mass of MAT sample.

The sample to be detected in step (2) above may be derived from a genetically engineered sample, a purified sample from a biological tissue cell, a biological fluid or a tissue culture fluid. The reaction time at 37° C. in step (3) above is 20 min, 30 min, 40 min, 50 min, 60 min, 70 min, 80 min, and 90 min.

Compared with the prior art, the beneficial advantages of the present invention are:
1. Make direct determination of the concentration of SAM possible instead of indirect determination of Pi, which directly reflects the MAT's capability of SAM synthesis, and the process is not affected by MAT configuration changes;
2. The SAM production and measurement in the present invention are done in one step in the same system. There is no lag, therefore the result is accurate and timely;
3. Immunoassays for SAM are not only specific but also very sensitive, and are not affected by the presentation form of SAM, which can be in both bound and dissociated forms;
4. Compared with the prior art, the present invention does not require special instruments. It only needs a conventional microplate reader.

Therefore, the use of the kit from the present invention makes the measurement of MAT activity more accurate, reliable, direct, simple and rapid, and can simultaneously obtain MAT activity and SAM concentration in a sample.

The great significance of the present invention lies in providing new MAT assay methods and products that allow researchers to conveniently and accurately determine MAT enzyme activity at any time. It plays important roles in understanding the expression profiles of MAT1A, MAT1B, MAT2 genes in different populations; any other in-depth understanding of the research and development work relating to methionine cycle; answering questions relevant to methionine metabolism; *in vivo* methylation status (methylation process); for the discussion of methylation mechanisms in various situations; in-depth studies of epigenetics; research and monitoring of metabolic diseases, oncogenesis, development, prognosis, nutrition, disease, and health. In addition, many metabolic processes such as homocysteine-cysteine-glutathione production (trans-sulfuration process) and polyamine (aminopropylation process) are also related to the production of SAM.

Another object of the present invention is to provide a methionine adenosyltransferase biological activity assay kit comprising the following components: a microplate coated with an anti-S-adenosylmethionine antibody or S-adenosylmethionine antigen or its analogues; tracer-labeled anti-S-adenosylmethionine antibody or tracer-labeled S-adenosylmethionine antigen or labeled S-adenosylmethionine analogues; S-adenosylmethionine standard; enzyme substrate solution consisting of methionine, adenosine triphosphate and a buffer of magnesium ions and potassium ions; a positive control (methionine adenosyltransferase); a tracer detection system; and an appropriate buffer system. Among them: microplates coated with S-adenosylmethionine antigen that can be in the form ofpolylysine (PLL), bovine serum albumin (BSA) or other carrier protein coupled S-adenosylmethionine or S-adenosylmethionine analogues, or directly coated onto the microplates with anti-S-adenosylmethionine antibodies (monoclonal and polyclonal) or indirectly coated through goat or rabbit anti-mouse IgG and then S-adenosylmethionine monoclonal antibody to the microplate; tracers may include enzymes, fluorescein, colloidal gold, chemiluminescent substances, biotin, digoxin (or digoxigenin), radioactive substances, various types of latex microspheres including fluorescent microspheres and colored Latex microspheres, etc.; display system corresponding to the tracer used; microplates are microplates consisting of removable ELISA strips; the substrate fluid and buffer system are determined after repeated tests to determine the optimal concentration of each component, pH and reaction time and so on.

The examples provided by the present invention are based on an enzyme-linked immunosorbent assay (ELISA), i.e. coated with S-adenosylmethionine antigen, or S-adenosylmethionine analogues; horseradish peroxidase or other tracer-labeled anti- SAM-specific antibody; S-adenosylmethionine or S-adenosylmethionine analogues as the standard; MAT positive control; substrate solution consisting of methionine, adenosine triphosphate, etc.; and proper buffer system; HRP substrate TMB (3,3',5,5'-tetramethylbenzidine) or other tracer display or detection system and ELISA stop solution. The amounts of methionine, adenosine triphosphate and methionine adenosyltransferase reported in the examples of the present invention are summarized here. The concentration of methionine and the concentration of adenosine triphosphate vary from micromolar to millimolar depending on the level of MAT activity tested. Because the substrate cross-reaction should be excluded and control is set for each experiment, in most cases excess substrate should bear no adverse effects. The pH of the enzyme reaction is 7.42-8.50. The concentration of MAT also varies depending on the source. The purified product of *E. coli* MAT gene expression used in this laboratory is employed at a concentration of 0.3-1.0 mg/ml. Suitable buffer systems include 50-100 mM magnesium ion, 50-400 mM potassium ion, 50-200 mM Tris-HCl buffer system.

### DETAILED DESCRIPTION OF THE INVENTION

Compared with the prior art, the key points of the present invention are:
1. One-step or simultaneous method, that is, the MAT enzymatic reaction and the quantification of the key product produced are performed together at the same time, or the MAT catalyzed chemical reaction is first performed for a period of time, about 20 min, which is very useful for the case when MAT activity is very low, and then the detection of product by immunological reaction is introduced. Since antigen-antibody binding takes a relatively longer time (it is generally assumed that 37°C for 1 h is needed), during the course of the immunological reaction, the MAT-catalyzed chemical reaction is still going on, i.e., the SAM is continuously generated. Therefore, regardless of whether the chemical reaction is first performed for a period of time alone or not, it is not contradictory to the key point in the present invention, i.e., the design in which the catalytic reaction and the immunological reaction are combined together. It is simply used in order to achieve the best detection sensitivity. For this purpose, (1) reflects the dynamic changes of MAT activity most precisely and without any delay; (2) Since the product SAM is very unstable, any SAM quantification methods, even though accurate, if they cannot measure the unstable SAM upon being produced, they are deemed to fail to calculate MAT enzyme activity efficiently, reliably and accurately.
2. Simultaneously measure the MAT activity and SAM concentration in the sample in the shortest possible time.
3. The combination of immunological methods and biochemical reactions enables substances in biochemical reactions to be detected efficiently, rapidly, specifically and sensitively.
4. The liquid formulation of the one-step method or the simultaneous method (that can sensitively reflect the reaction that changes the MAT activity) not only ensures that the enzymatically catalyzed biochemical reaction can proceed normally, but also enables the immunoassay step to be effectively implemented.

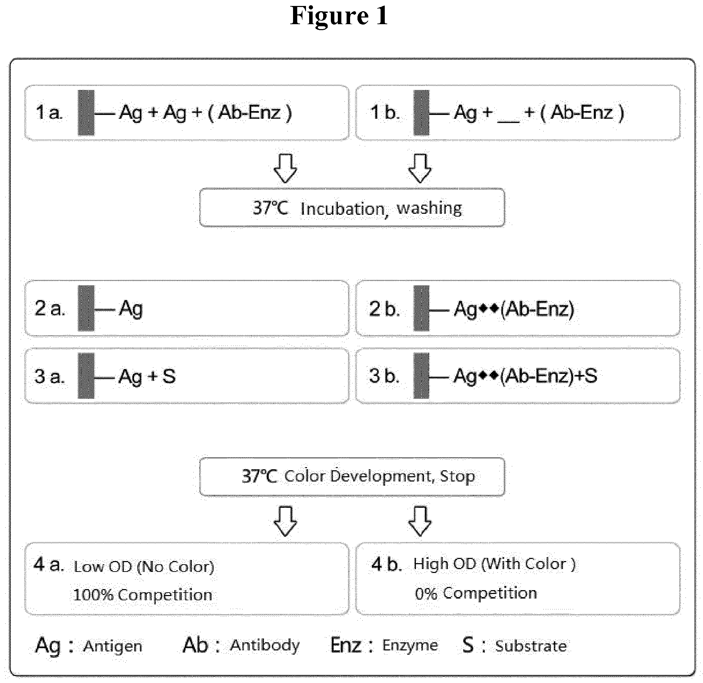

The principle of the direct *in vitro* determination of MAT activity of the present invention is summarized as follows: An enzyme-linked plate coated with SAM antigen is added with an appropriate amount of HRP-labeled anti-SAM antibody. Then, a SAM standard or a MAT activity assay system (a suitable buffer containing methionine and adenosine triphosphate, plus a MAT positive control or a sample having MAT activity to be measured) was added as a SAM test sample. Incubate at 37 °C for a certain period of time, then add HRP substrate TMB to develop color, and read the optical density at 450 nm. Based on the standard curve of SAM, the concentration of SAM can be calculated. Based to the amount of SAM, the ability of MAT to produce SAM can be calculated. MAT samples can be derived from genetically engineered MAT, purified tissue samples from biological tissue cells, MAT within tissues and cells, biological or tissue cell culture fluids. Examples of biological fluids are blood, plasma, serum, saliva, urine, cerebrospinal fluid, abdominal or thoracic exudate, tissue fluid, etc.
The detailed technical principle and method of this invention is as follows:
1. MAT catalytic reaction principle: 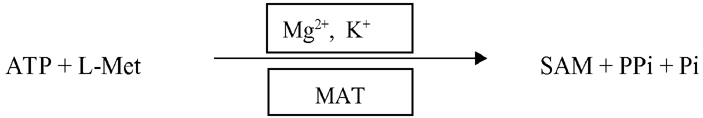
2. Principle and method of direct competitive ELISA measurement:
   (A) Antigen-coating method: A PLL or BSA-conjugated SAM antigen is pre-coated on a solid-phase ELISA micro-titer plate that is then blocked with BSA solution. The antigen to be detected (SAM sample), then HRP-conjugated anti-SAM antibody are added and incubated at the same time. The antigen to be tested and the coating antigen compete for the enzyme-labeled antibody. After incubation and washing, the enzyme-labeled antibody bound to the coating antigen is retained, and the enzyme-labeled antibody that binds to the antigen to be detected (free antigen) is washed away. Finally, adding TMB substrate to the reaction wells. The final coloration result is inversely proportional to the amount of the antigen to be detected, that is, the higher the OD450 value, the lower the SAM concentration in the sample to be measured. In the course of the experiment, a series of SAM solutions with a known concentration were used as standards in the same ELISA micro-titer plate for the standard curve of the assay. Based on the standard curve, an equation showing how OD450 readings and values of the known standard concentration are related can be obtained. Put the sample OD450 into the equation to get the corresponding concentration value of the sample. The principle of this direct competitive ELISA is shown in Figure 1.
   (B) Antibody-coating method: First coat goat or rabbit anti-mouse IgG onto micro-titer plate at 4°C, overnight, add the proper amount of anti-SAM antibody to the wells and incubate for 30-60 min, or directly coat the optimal amount of anti-SAM antibody to the micro-titer plate. Wash plate, add HRP conjugated SAM antigen or its analogues, then add SAM standard and samples. The standards and samples compete with HRP conjugated SAM antigen or its analogues to bind anti-SAM specific antibody on the plate. Incubate for 30 min and then wash plate. Add TMB substrate and incubate for 15 min, then add stop solution. Record OD450. According to the standard curve, the concentration of SAM from the samples can be calculated.
3. Combine the catalytic reaction of the MAT and the direct competitive ELISA to investigate the feasibility of a one-step method or a simultaneous method. In order to make it feasible and workable, the present invention is divided into the following parts to elaborate the technical details of the present invention:
   Part 1: Exclude the existence of cross-reaction between anti-SAM antibody and methionine adenosyltransferase substrate ATP and L-Met in order to avoid interference of cross-reaction to the competitive ELISA.
   Part 2: Explore the optimal reaction ratio between methionine adenosyltransferase and its substrate ATP and L-Met under different pH values.
   Part 3: Determine the optimal reaction time of MAT and set the appropriate time to measure the amount of SAM synthesis reaction. Plot a curve showing SAM concentration over time. The reaction of various anti-SAM monoclonal antibodies 118-6, 84-3 and rabbit anti-SAM polyclonal antibodies with enzymatically synthesized SAM are confirmed.
   Part 4: Determine the enzymatic activity of MAT purified from mouse liver cells under optimal reaction conditions.
   Part 5: Under optimal reaction conditions, measure MAT enzymatic activity in normal liver cell line L02 and hepatoma cell line HepG2 under different MAT regulatory factors, and observe the changes.
   Part 6: Experiment with different coating antigens and SAM standards to determine their impacts on MAT activity under the determined optimal reaction conditions.
4. Repeated tests are done to optimize the optimum concentration of each component, reaction time, buffer formulation and pH value, standard curve and the range of standard concentrations.

The above technical route and methods have been fully confirmed by the following examples of the invention. It is apparent from the above, that the advantages of the method of the present invention include, but are not limited to the following: (1) Direct determination of the SAM, the key product of the MAT catalyzed reaction, but not of the secondary product Pi, and thus the most direct reflection of the MAT's ability to synthesize SAM; (2) SAM production and measurement are performed in one step at the same time in the same reaction and incubation system, with which the changes of MAT activity under different conditions can be captured timely and accurately and reaction time of the enzyme can be controlled better. Therefore, the results will be very accurate and timely; (3) Using anti-SAM antibodies with very high specificity, sensitivity and affinity and measure it timely, the results are not only specific, but also very sensitive and are not influenced by the different forms of SAM (binding and dissociative); (4) Being convenient, direct, fast, and therefore very useful in studying the effects of various factors on the activities of MAT from samples. Even if the influence was subtle, it could be identified and captured.

The present invention also studied BSA and hemocyanin (KLH) conjugated SAM or SAM antigen analogues and used them to coat micro-titer plates as in the 2 (A) Antigen-coating method above; and a direct method competitive ELISA for the measurement of SAM as in the 2 (B) Antibody-coating method, the results were consistent with those described in the Examples..

### DESCRIPTION OF THE DRAWINGS

In order to clearly describe the technical solutions in the embodiments of the present invention or in the prior art, the drawings used in the description of the embodiments or the prior art are briefly described below.
Figure 1 is a schematic of a direct competitive ELISA steps and components. The figure shows an example of an antigen instead of an antibody bound to the plate.

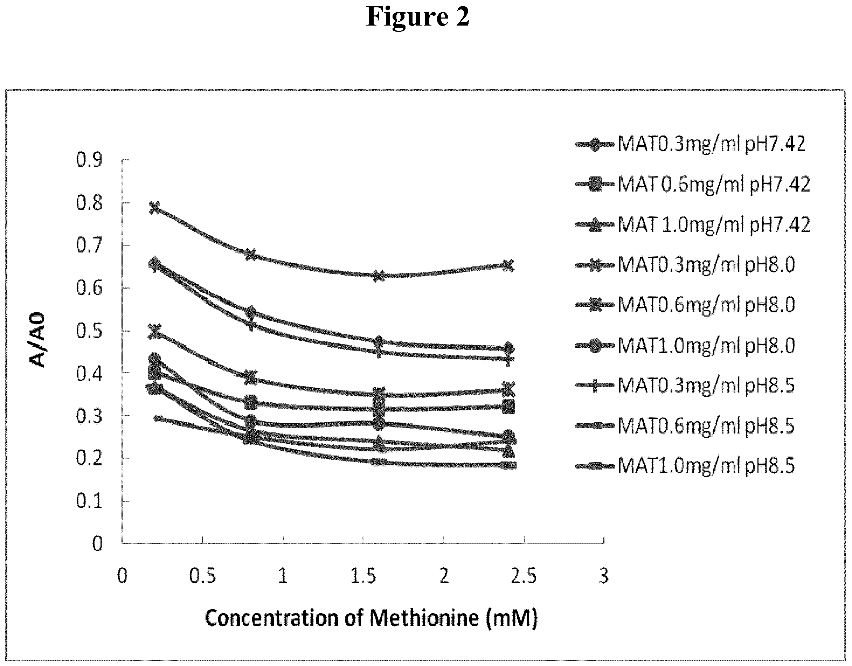

Figure 2 illustrates the effects of methionine adenosyltransferase concentration, amount of methionine substrates, and pH of the reaction buffer on the synthesis of S-adenosylmethionine. The synthesis yield of SAM was expressed as A/A0, i.e., the ratio of sample well OD450 to control well OD450; the lower the A/A0, the greater the ability of the samples or standards to compete with the coating antigen for HRP-labeled antibody. The higher the SAM production, the greater is the MAT activity.

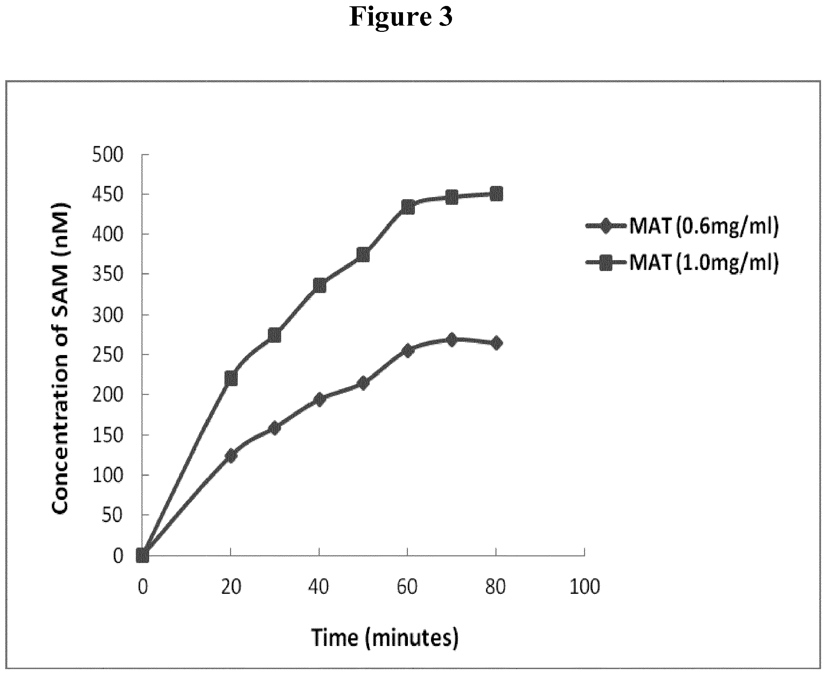

Figure 3 shows the determination of the optimal reaction time in the direct determination of MAT activity *in vitro.* MAT enzyme catalyzed the substrates to generate SAM in a straight rising phase before 60 min; then within 60-90 min, the SAM content did not change significantly and was in the plateau phase, indicating that the concentration of substrates might be insufficient or the enzyme activity might be lost after 1 h, leading to no more SAM production. The reaction was faster when the enzyme concentration was 1.0 mg/ml rather than 0.6 mg/ml.

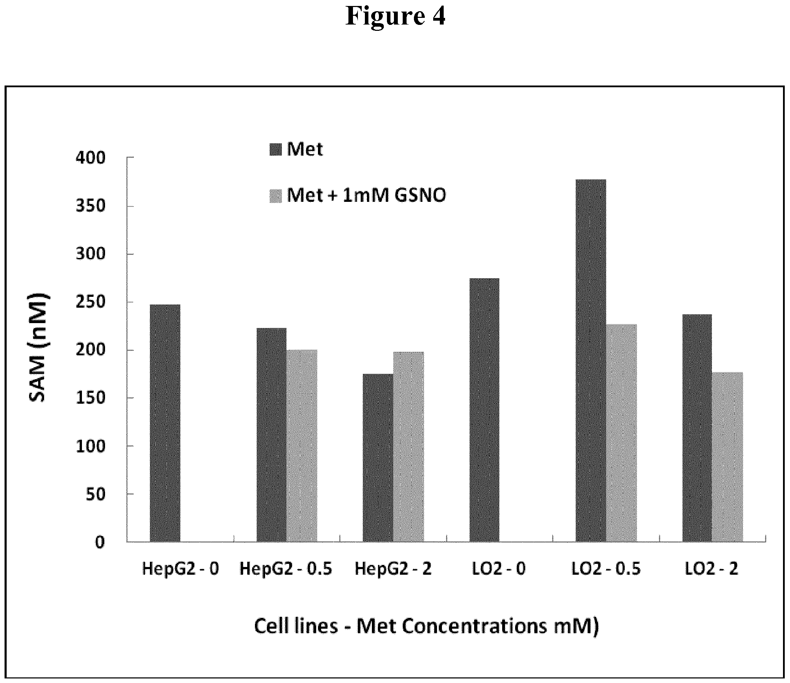

Figure 4 describes the regulation of MAT activities by nitrosoglutathione (GSNO) and Met in normal liver cell line L02 and liver cancer cell line HepG2. The ordinate is the SAM concentration by MAT catalyzed synthesis. The activities of MAT-I/III in L02 cells and MAT-II in HepG2 cells were regulated by GSNO and different concentrations of Met. For L02 cells, 500 µM Met stimulated MAT-III/I activity, 2 mM Met stimulation was not significant. The inhibitory effect of GSNO nitrosylation on MAT-III/I was obvious. In HepG2 cells, Met inhibits MAT-II activity, and GSNO has no inhibitory effect on MAT-II.

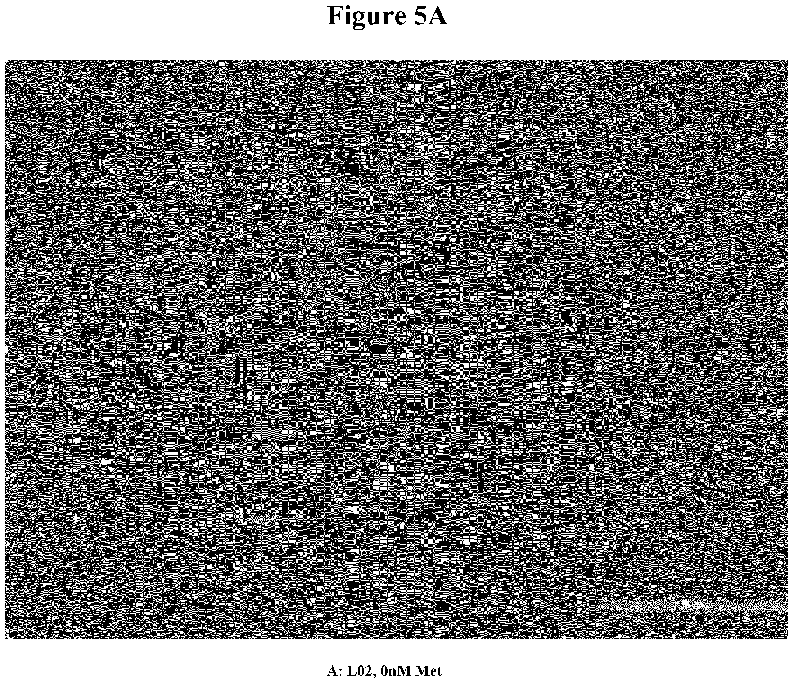

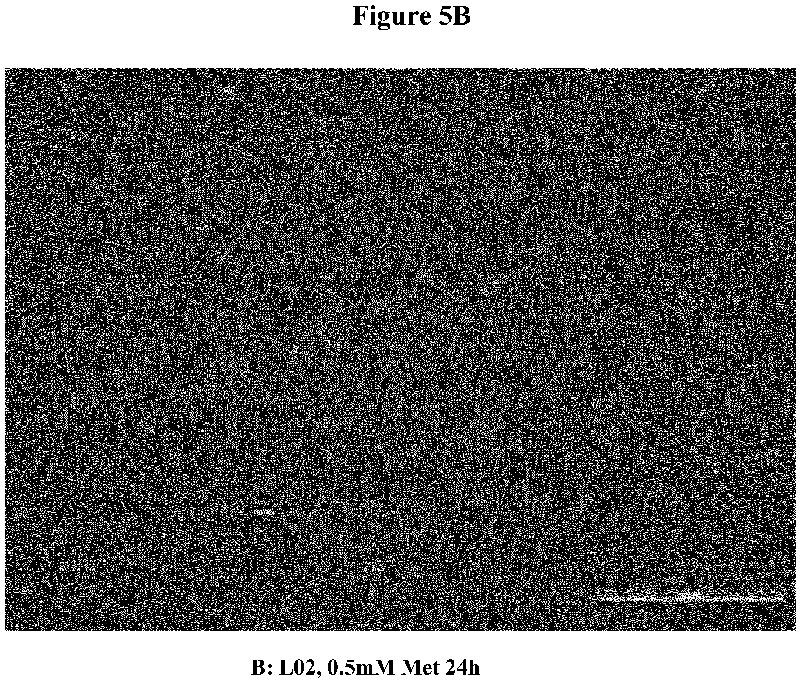

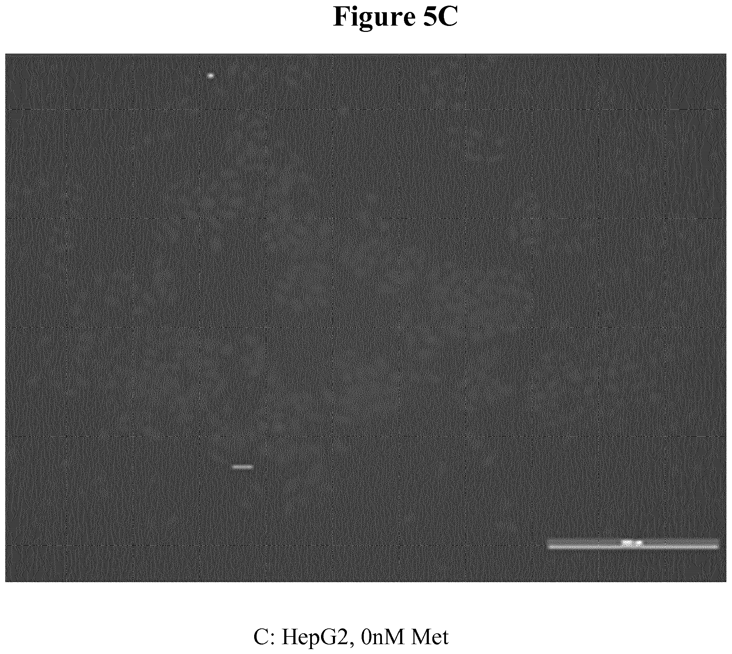

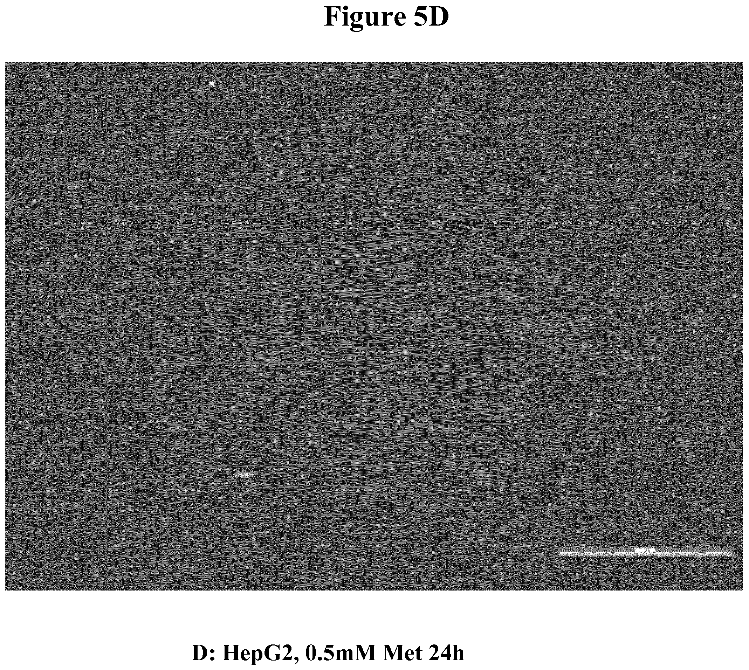

Figures 5A to Figures 5D show a comparison of the results of immunofluorescence staining using anti-SAM monoclonal antibody at 1:400 dilution before and after stimulation with 0.5 mM Met for 24 h on normal hepatocyte L02 and hepatocellular carcinoma HepG2 (x200). Figure 5A features the results for L02, 0 nM Met; while Figure 5B describes the results for L02, 0.5 mM Met 24 h. Figure 5C shows the results for HepG2, 0 nM Met; while Figure 5D shows results for HepG2, 0.5 mM Met 24 h. On a black background, green fluorescent (light-colored) positive cells showed SAM expression, and black background showed no or very low SAM. It can be seen that Met stimulated MAT-III/I activity of L02 cells, and thus SAM expression increased in group shown in Figure 5B compared to group shown in Figure 5A; conversely, Met inhibited the activity of MAT-II in HepG2 cells, and thus SAM expression in group of Figure 5D was lower than in the Figure 5C group.

### DETAILED DESCRIPTION OF THE INVENTION

The following further describes the present invention in detail with reference to the experimental results and data. Raw materials not mentioned are conventional commercial reagents
and are commercially available.

### EXAMPLE 1

### Cross-reactivity of SAM and methionine adenosyltransferase substrates ATP and L-Met Experimental Materials

Anti-SAM and HRP-conjugated anti-SAM antibody: Arthus Biosystems, MA00201, MA00202, PA00201, MAH00201; polylysine (PLL) or bovine serum albumin (BSA) conjugated SAM antigens (for coating ELISA plates): Arthus Biosystems, ACT00201, ACT00204; S-adenosylmethionine (aza-SAM) standard: Arthus Biosystems, AST00201; S-adenosylmethionine (adenosine methionine disulfide methionine, SAMe) standards: Sigma, A2408, and the same named purchased from Shaanxi Pioneer Biotechnology Co., Ltd.; Methionine adenosyltransferase (MAT): Beijing Aibixin Biotech Co., Ltd. Abt-P-005; BSA, Tris, also known as tris (hydroxymethyl) aminomethane, NaCl, NaH₂PO₄, Na₂HPO₄: Beijing Huameijiacheng Biotechnology Co., Ltd.; KCl: Tianjin Damao Chemical Reagents Co. Ltd.; MgSO₄: Hunan Honghao Gene Technology Co., Ltd.; Adenosine triphosphate (ATP): Shanghai Jingchun Chemical Reagents Co., Ltd.; L-Met, ProClin 300: Sigma; TMB: Huzhou Yingchuang Biotechnology Co., Ltd.; Sulfuric acid: Hunan Kangdu Pharmaceutical Co., Ltd.; 96-well enzyme-linked immunoassay micro-titer plate: US Corning high adsorption enzyme-linked immunoassay plate.

### Reagent preparation

Enzyme Reaction Buffer: 100 mM Tris, 100 mM KCl, 20 mM MgSO₄, ProClin 1%, adjusted pH values pH 7.42, pH 8.0, pH 8.5 respectively; SAM analogue standard (aza-SAM): 960 nM, 480 nM, 240 nM, 120 nM, 60 nM , 30nM, 15nM, 0nM, in enzyme reaction buffer pH7.42; SAM analogous quality control: 200nM aza-SAM in enzyme reaction buffer pH 7.42; SAM standard (SAMe): 1-40 µM in enzyme reaction buffer pH7.42; SAM quality control in: 8µM SAMe in enzyme reaction buffer pH7.42; the enzyme reaction buffer: 0.5% or 0.2% IB (Incubation Buffer): 10nM PB (Phosphate Buffer), 150 mM NaCl, 0.5% or 0.2% BSA, 0.1% ProClin.

The ELISA plates were coated with PLL_SAM antigen. The SAM standard curve was created using 0, 15, 30, 60, 120, 240, and 480 nM standards IB containing 0.5% BSA. The concentrations of the cross-reacting substances ATP and L-Met were 0, 120, 1200, 3960 and 12000nM. The HRP-anti-SAM antibody was diluted at 1:25000 with HRP antibody diluent. Added 30 µl standards and cross-reacting substances ATP and Met per well, and 70 µl diluted HRP-anti-SAM antibody per well and incubated at 37°C for 1h. Washed the plate, added 100 µl TMB at 37°C for 15min, added 50 µl stop solution and read OD450. The results are shown in Table 1.

**Table 1 Reaction rates of anti-SAM monoclonal antibodies with ADP and Met (A/A0)**

| **SAM nM** | **SAM standard** | **ATP** | **L-Met** | **nM** | **Cross-reaction (%)** |
|---|---|---|---|---|---|
| 0 | 1 | 1 | 1 | 0 | |
| 15 | 0.793999439 | 1.0513698 | 1.0421467 | 120 | 50% |
| 30 | 0.723637723 | 1.0007116 | 1.0464435 | 1200 | 10% |
| 60 | 0.585606131 | 1.094967 | 1.0118777 | 3960 | 3% |
| 120 | 0.430096271 | 1.0861819 | 1.0953228 | 12000 | 1% |
| 240 | 0.271109449 | | | | |
| 480 | 0.123357323 | | | | |
| Cross-reaction (%) | | <1% | <1% | | |

| | | | | | |
|---|---|---|---|---|---|
| A/A0; reaction rate (A0 is the OD450 reading at 0 nM, A represents any OD450 reading of wells that have different amount of free small molecule antigen competing.) Cross-reaction rate = (Concentration of free antigen when 50% inhibition is achieved/ Concentration of cross-reaction substances when 50% inhibition is achieved) *100%. The results of repeated experiments showed that the cross-reaction rates of ATP, ADP and Met with anti-SAM monoclonal antibodies 118-6, 84-3 and rabbit anti-SAM polyclonal antibody. R3 were much less than 1%. The cross-reaction of anti-SAM antibody with methionine, adenosine, S-adenosylhomocysteine and methylthioadenosine are all far less than 1% (see antibody product data from Arthus Biosystems). Therefore, this experiment does not have any cross-reaction with other components in the reaction system. | | | | | |

### EXAMPLE 2

### Enzyme activity assay system: optimum reaction ratio of methionine adenosyltransferase, methionine and adenosine triphosphate under different pH conditions

Using ELISA strips that were pre-coated with PLL-SAM antigen, and different concentrations of ATP, Met, and MAT enzyme were formulated with the enzyme reaction buffer at pH 7.42, pH 8.0, and pH 8.5 respectively. As shown below, codes A\B\C\D represents four Met concentrations, adenosine triphosphate components, and code 1\2\3 represents three MAT concentrations. The combination of the two codes was used to prepare the chessboard design. A total of 12 different combinations of methionine adenosyltransferase, methionine, adenosine triphosphate solutions have been obtained.

| Code | ATP | Met |
|---|---|---|
| A | 2.4mM | 0.2mM |
| B | 2.4mM | 0.8mM |
| C | 2.4mM | 1.6mM |
| D | 2.4mM | 2.4mM |

| Code | MAT |
|---|---|
| 1 | 0.3mg/ml |
| 2 | 0.6mg/ml |
| 3 | 1mg/ml |

The reaction was performed at 37°C for 1 hour. After the plate was washed, 100 µL of TMB was added to each well. After incubation at 37°C for 15 minutes, 50 µl of the stop solution was added before reading OD450. The results are shown in Table 2 below.

**Table 2 Comparison of OD450 values from MAT-catalyzed synthesis reactions under different substrate concentrations, MAT levels and pH conditions**

| pH=7.42 | | | | | | | |
|---|---|---|---|---|---|---|---|
| OD450 | | | | design | | | |
| 1.9715 | 1.8155 | 1.0839 | 1.0683 | A1 | A1 | A3 | A3 |
| 1.6422 | 1.4980 | 0.8157 | 0.7668 | B1 | B1 | B3 | B3 |
| 1.4120 | 1.3400 | 0.7347 | 0.7082 | C1 | C1 | C3 | C3 |
| 1.3852 | 1.2713 | 0.6608 | 0.6588 | D1 | D1 | D3 | D3 |
| 1.2270 | 1.1315 | 2.6735 | 2.9059 | A2 | A2 | A background | 0 competition pH=7.42 |
| 1.0224 | 0.9309 | 2.6567 | 2.7983 | B2 | B2 | B background | 0 competition pH=7.42 |
| 0.9868 | 0.8753 | 2.7033 | 0.0488 | C2 | C2 | C background | blank |
| 0.9889 | 0.9092 | 2.8659 | 0.0470 | D2 | D2 | D background | blank |

| pH=8.0 | | | | | | | |
|---|---|---|---|---|---|---|---|
| OD450 | | | | design | | | |
| 2.1943 | 2.1483 | 1.1701 | 1.2561 | A1 | A1 | A3 | A3 |
| 1.8445 | 1.9023 | 0.8043 | 0.8439 | B1 | B1 | B3 | B3 |
| 1.7334 | 1.7538 | 0.7860 | 0.8290 | C1 | C1 | C3 | C3 |
| 1.7352 | 1.8867 | 0.7320 | 0.7191 | D1 | D1 | D3 | D3 |
| 1.4195 | 1.3596 | 2.4978 | 2.7377 | A2 | A2 | A background | 0 competition pH=8.0 |
| 1.1351 | 1.0597 | 2.5387 | 2.7492 | B2 | B2 | B background | 0 competition pH=8.0 |
| 0.9960 | 0.9884 | 2.5973 | 0.0487 | C2 | C2 | C background | blank |
| 1.0172 | 1.0253 | 2.7131 | 0.0461 | D2 | D2 | D background | blank |

| pH=8.5 | | | | | | | |
|---|---|---|---|---|---|---|---|
| OD450 | | | | Design | | | |
| 1.6684 | 1.9050 | 1.0299 | 1.0203 | A1 | A1 | A3 | A3 |
| 1.2567 | 1.5798 | 0.6783 | 0.7050 | B1 | B1 | B3 | B3 |
| 1.1811 | 1.3139 | 0.5387 | 0.5755 | C1 | C1 | C3 | C3 |
| 1.1386 | 1.2621 | 0.5609 | 0.5186 | D1 | D1 | D3 | D3 |
| 0.8358 | 0.8323 | 2.4289 | 2.7261 | A2 | A2 | A background | 0 competition pH=8.5 |
| 0.6387 | 0.8024 | 2.5742 | 2.7016 | B2 | B2 | B background | 0 competition pH = 8.5 |
| 0.6104 | 0.6657 | 2.5285 | 0.0468 | C2 | C2 | C background | blank |
| 0.6390 | 0.7459 | 2.6965 | 0.0484 | D2 | D2 | D background | blank |

The synthetic yield of SAM is expressed as A/A0, i.e. the ratio of OD450 of sample well to control well. The lower the A/A0, the greater the ability of the SAM synthesized to compete with the coating antigen for HRP-labeled antibodies. The higher the SAM production, the greater is the MAT activity. The results are shown in Table 3 below.

**Table 3 Comparison of relative amounts of SAM catalyzed by MAT under different substrate concentrations, MAT levels and pH conditions**

| A/A0 pH=7.42 | | | | | |
|---|---|---|---|---|---|
| | MTA 0.3 | | MTA 0.6 | | MTA 1.0 |
| A1 | 0.658156 | A2 | 0.403448 | A3 | 0.366664 |
| B1 | 0.542829 | B2 | 0.3312 | B3 | 0.265085 |
| C1 | 0.473611 | C2 | 0.314938 | C3 | 0.240193 |
| D1 | 0.456583 | D2 | 0.321357 | D3 | 0.218208 |

| A/A0 pH=8.0 | | | | | |
|---|---|---|---|---|---|
| | MTA 0.3 | | MTA 0.6 | | MTA 1.0 |
| A1 | 0.787768 | A2 | 0.497812 | A3 | 0.432365 |
| B1 | 0.677275 | B2 | 0.389451 | B3 | 0.288083 |
| C1 | 0.629131 | C2 | 0.350432 | C3 | 0.281926 |
| D1 | 0.654111 | D2 | 0.361207 | D3 | 0.25153 |

| A/A0 pH=8.5 | | | | | |
|---|---|---|---|---|---|
| | MTA 0.3 | | MTA 0.6 | | MTA 1.0 |
| A1 | 0.652252 | A2 | 0.294959 | A3 | 0.366613 |
| B1 | 0.514064 | B2 | 0.252391 | B3 | 0.241552 |
| C1 | 0.450024 | C2 | 0.221449 | C3 | 0.191089 |
| D1 | 0.432341 | D2 | 0.241852 | D3 | 0.184582 |

The resulting reaction rate, i.e. the A/A0 and methionine concentrations were plotted to obtain the results shown in Figure 2. (1) At different pH conditions, when the amounts of substrate and enzyme are constant, the A/A0 at pH 8.5 is minimized and is the highest at pH 8.0. However, the production of SAM increase was not very significant; (2) when the substrate ATP was unchanged (2.4 mM), Met increased from 0.2, 0.8, 1.6, to 2.4 mM, the SAM production increased significantly; (3) When the amount of MAT enzyme increased, its SAM production increased rapidly.

Based on the results from three different conditions above, the strongest factor influencing the SAM formation reaction is the amount of MAT, followed by the increase in substrate Met, and the smallest factor is the buffer pH value. In addition, because the MAT used in this experiment is a product of *E. coli* MAT gene expression, it's catalytic activity might not be ideal, therefore the required substrate concentration was relatively higher, but the experiment shows that even in the milli-molar level of Met and ATP, there was no cross-reaction. Since the aim of this experiment was not to screen for highly active MAT, the MAT selected here is able to meet the needs of establishing a MAT activity determination methods. Due to the relatively high activity of MAT under alkaline conditions such as pH 8.5, the antigen-antibody binding is reduced at pH 8.5 compared to pH 7.42, thus A/A0 from Figure 2 appeared relatively low at pH 7.42 and pH 8.5 (i.e, the amount of SAM synthesis was high), and the A/A0 was relatively high at pH 8.0 (i.e, the amount of SAM synthesis was small). Therefore, the best MAT measurement system should take into account various factors. The pH value is set to be either 7.42 or 8.5.

### EXAMPLE 3

### Determination of the optimal reaction time for measurement of MAT activity in vitro

Take PLL-SAM-coated antigen ELSIA strips, and add SAM standards at concentration of 0, 15, 30, 60, 120, 240, 480, 960 nM, and quality control 200 nM that were prepared in an enzyme reaction buffer at pH 7.42. Add ATP and Met at 2.4mM, plus MAT enzyme at 0.6mg/ml, or ATP and Met at 2.4mM, plus MAT at 1.0mg/ml (all final concentrations) as SAM substrate and enzyme mixture. Add 30 µl per standard well and enzyme samples, and 70 µl per well of HRP-anti-SAM antibody. Then incubated at 37 °C for 30min, 60min, 90min, after washing the plate, 100 µl TMB was added and left at 37 °C for 15min, added 50 µl stop solution and read OD450. Results are shown in Table 4.

**Table 4 Effect of different reaction times on the amount of SAM catalyzed by MAT**

| Concentration (nM) | 30 min | 60 min | 90 min |
|---|---|---|---|
| ATP and Met 2.4mM, MAT 0.6 mg/ml | 116.3030073 | 221.5053 | 204.34158 |
| ATP and Met2.4mM , MAT 1.0 mg/ml | 270.8731347 | 396.8892 | 410.82783 |

The results above indicated that the amount of SAM synthesized at 60 minutes was significantly higher than that at 30 minutes, but was almost the same at 90 minutes and 60 minutes. Measured SAM synthesis from the 20-90 minutes time period, the relationship of SAM production change over time could be obtained.

Take PLL-SAM-coated antigen ELSIA strips, and add SAM standards and quality control standards were prepared in an enzyme reaction buffer at pH 7.42, added ATP and Met at 2.4mM, plus MAT enzyme at 0.6mg/ml, or ATP and Met at 2.4mM, plus MAT at 1.0mg/ml (all final concentrations) as SAM substrate and enzyme mixture. Added 30 µl per standard well and enzyme samples, and 70 µl per well of HRP-anti-SAM antibody. Incubation time at 37 °C is shown in Table 5. After washing the plate, 100 µl TMB was added and left at 37 °C for 15min, added 50 µl stop solution and read at OD450. Data processing is the same as done previously. The concentrations measured are as follows.

**Table 5 Relationship between reaction time and SAM synthesis**

| | SAM yield (nM) over time | | | | | | |
|---|---|---|---|---|---|---|---|
| Reaction Conditions | 20 min | 30 min | 40 min | 50 min | 60 min | 70 min | 80 min |
| ATP and Met (2.4mM), MAT (0.6mg/ml) | 124.28 | 158.98 | 194.17 | 215.13 | 255.39 | 268.26 | 264.06 |
| ATP and Met (2.4mM), MAT (1.0mg/ml) | 220.63 | 274.60 | 335.53 | 374.90 | 433.83 | 445.82 | 450.21 |

| | The corresponding MAT activity (U/mg) | | | | | | |
|---|---|---|---|---|---|---|---|
| ATP and Met (2.4mM), MAT (0.6mg/ml) | 10.35667 | 8.832095 | 8.090302 | 5.837829 | 7.094171 | 6.38704 | 5.188694 |
| ATP and Met (2.4mM), MAT (1.0mg/ml) | 11.03143 | 9.153337 | 8.388306 | 7.298031 | 7.647138 | 6.225992 | 5.627597 |

The concentration of synthesized SAM and reaction time were plotted and the results are shown in Figure 3. The experimental results have been verified several times under different buffer systems and different MAT conditions. The results showed that the amount of SAM synthesized by MAT was in a straight line rising phase before 60 min, and the amount of SAM did not change significantly during the period of 60-80 min, i.e. in a plateau phase, indicating that the concentration of substrate might not be enough or the enzyme activity was lost after 1 h, resulting in no increase in SAM production. The reaction rate was faster when the enzyme concentration was 1.0 mg/ml rather than 0.6 mg/ml.

### EXAMPLE 4

### The optimal buffer system for determination of MAT activity in vitro

Using a system similar to the example above, the coating PLL-aza-SAM was used at 0.05 µg/ml. The HRP-anti-SAM antibody 118-6 was diluted at 1:30000 and was incubated for 1 hour. The pH value of the prepared solution was 7.40±0.05, and the ATP was at 5 mM, Met at 4 mM, MAT at 1 mg/ml. When titrating the concentration of Mg²⁺, the buffer contained 250 mM KCl and 100 mM Tris. When titrating the concentration of K⁺, the buffer contained 20 mM MgSO₄ and 100 mM Tris. After the optimal concentrations of Mg²⁺ and K⁺ were determined, Tris was titrated again. The results are shown in Table 6. The A/A0 was gradually decreased by 0.475 when the concentration of MgSO₄ was from 4 mM to 100 mM. However, A/A0 decreased rapidly when the concentration of MgSO₄ was from 4 to 50 mM, and decreased slowly when the concentration of MgSO₄ was from 50 to 100 mM. Thus, the suitable MgSO₄ concentration was selected between 50 mM and 100 mM. The A/A0 has small changes when the concentration of KCl was from 50-400 mM, There was no significant decrease and increase trend, and chose about 150mM. The concentration of Tris was 100 mM and remained optimal.

**Table 6 Relationship between reaction time and SAM synthesis**

| MgSO₄ | | KCl | | Tris | |
|---|---|---|---|---|---|
| Conc. (mM) | A/A0 | Conc. (mM) | A/A0 | Conc. (mM) | A/A0 |
| 4 | 0.2199 | 50 | 0.1983 | 20 | 0.4981 |
| 10 | 0.2047 | 100 | 0.1938 | 50 | 0.2567 |
| 30 | 0.1867 | 150 | 0.1911 | 100 | 0.2168 |
| 50 | 0.1766 | 200 | 0.1973 | 200 | 0.2472 |
| 70 | 0.1734 | 250 | 0.2079 | | |
| 100 | 0.1724 | 300 | 0.2106 | | |
| | | 400 | 0.1995 | | |

### EXAMPLE 5

### Determine the enzymatic activity of MAT purified from mouse liver cells under optimal reaction conditions

### Experimental Materials

Sterile PBS, 0.25% trypsin (Aladdin), 1640 medium (Gibco), 10% FBS (Yuanheng St. Ma), serum-free MEM medium (Gibco), 10 g/ml Met aseptic concentrate, GSNO, 15 cm² square bottle, 75 cm² square bottle (Corning), FITC- goat anti-mouse IgG (abcam), DAPI (Life Technologies); Triphenylene blue powder (Aladdin).

Phosphate buffer (PBS): NaCl 8g, Na₂HPO_{4.12}H₂O 2.885g, KCl 0.2g, KH₂PO₄ 0.2g, ultrapure water 1000 ml; trypsin (0.25% trypsin): trypsin 0.1g, 4% EDTA solution 200µl 39.8 ml of 1×PBS solution; Trypsin is fully dissolved and filtered with a 0.2 µm sterile filter (Pall). Trypan blue solution (4% Triphenyl blue solution): Triphenyl blue 1.6 g, ultrapure water 40 ml, filtered with filter paper.
The experimental design is shown in Table 7.

**Table 7 Experimental group parameters of GSNO pretreatment and Met on the intracellular MAT activity in L02 and HepG2 cells**

| Number | 1 | 2 | 3 | 4 | 5 |
|---|---|---|---|---|---|
| GSNO | 0mM | 0mM | 0mM | 1mM | 1mM |
| Met | 0mM | 0.5mM | 2mM | 0.5mM | 2mM |

### Experimental steps:

1. When the cells in the flask are at about 80% confluence, remove the 1640 medium (with 10% fetal bovine serum) and wash with PBS once;
2. Take the two groups containing GSNO, treat the cells with serum-free MEM medium containing 1 mM GSNO and leave in the incubator for 30 min.
3. Remove the serum-free medium containing GSNO and wash it with PBS once;
4. Add Met-containing serum-free MEM medium (containing 5% fetal bovine serum and 2 mM glutamine) into the square flask according to the parameters in the table above. Add the Met-free MEM as the control group and put into the incubator for 24h;
5. Remove the culture medium from the flask and add an appropriate volume of trypsin to ensure that the pancreatin covers the bottom of the entire square flask;
6. Place the flask in a 37°C, 5% CO₂ incubator for 2-3 minutes. When cells start to fall out, add 5 ml of 1640 medium containing 10% FBS to terminate the action of pancreatin.
7. Resuspend with about 1 ml of PBS after centrifugation, and count with trypan blue staining.
8. Take 1 ml of each cell suspension, place on an ice bath and sonicate it (see Example 5). Centrifuge at 15000 g and then collect the supernatant.
9. Reaction is performed as described in Example 3 at 37°C for 60 min to determine the MAT activity in the cell supernatant.

Since the amount of cells in each experimental group used for the ELISA assay was not exactly the same, L02, HepG2 cells were normalized by cell count (adjusted to 2x10⁷). The amounts of SAM synthesized by MAT under different conditions are shown in Table 8 and Figure. 4.

**Table 8 Effects of GSNO and Met on different MAT activities in the L02 and HepG2 cells**

| HepG2 | SAM (nM) | L02 | SAM (nM) |
|---|---|---|---|
| 0mM Met | 247.4761 | 0mM Met | 274.4578162 |
| 0.5mM Met | 222.3993 | 0.5mM Met | 377.700459 |
| 2mM Met | 174.3621 | 2mM Met | 236.4367472 |
| 0.5mM Met+1mM GSNO | 200.6807 | 0.5mM Met+1mM GSNO | 226.6779748 |
| 2mM Met+1mM GSNO | 198.1654 | 2mM Met+1mM GSNO | 176.6032683 |

Human normal liver cell line L02 expresses the dimer MAT-III or tetramer MAT-I consisting of the catalytic subunit α1 encoded by the MAT1a gene, whereas HepG2 hepatoma cells express tetramer MAT-II consisting of the catalytic subunit α2 and regulatory subunit β encoded by the MAT2a/2b gene. The responses to Met stimulation, cell types of expression and effects of the two MAT enzymes encoded by these two genes are not the same. The presence of MAT-III or MAT-I in adult hepatocytes is sensitive to Met stimulation, and its function is to rapidly reduce Met levels in the blood during high Met diets. Therefore, MAT-III or MAT-I activity is increased by Met stimulation [5]. Our results show that 500 µM Met stimulated the activity of MAT-III or MAT-I *in vitro* for 24 hours, and the inhibition of MAT-III or MAT-I after nitrosation of GSNO was obvious. No stimulatory effects of Met at 2 mM dosage on MAT-III or MAT-I activity was observed, and MAT activity on the contrary was slightly decreased. The reason was unclear. However, GSNO nitrosylation-inhibitory effects on MAT activity in the presence of 2 mM Met remained obvious. In contrast, the dose-response relationship of Met inhibits MAT-II activity in hepatoma cell lines is consistent with the literature [12], i.e. the higher the Met concentration, the lower the activity of MAT-II. In addition, our results also showed that nitrosylation has a minor effect on MAT-II, which is in agreement with the reported elsewhere that NO in GSNO inhibits MAT activity by binding to the cysteine at position 121 of MAT-II [5]. GSNO mainly inhibits the activity of MAT-III/I post Met stimulation.

Immunofluorescence staining of these two cell lines was performed simultaneously with the competitive ELISA described above for quantification of SAM, the procedure is as follows:
(1) Digest the HepG2 and L02 cells from the bottom of the square flask;
(2) Centrifuge at 1050 rpm for 5 min and re-suspend the cells in 1640 medium containing 10% FBS in (Gibco);
(3) After counting with trypan blue, cells were seeded onto 24 wells at a density of 7.5 x 10⁴ cells/well, and 8 wells were seeded each cell line;
(4) Add the medium to 24-well culture plate to 1 ml/well and place it in the incubator for 24 h;
(5) Remove the original culture medium from the wells. Add 1 ml/well MEM medium containing 5% FBS and 500 µM Met according to Table 1, and put the culture plate into the incubator for 24 h.
(6) After 24 h. discard the medium and wash it twice with 37 °C pre-warmed PBS. Add 500 µl 80% ice acetone per well at -20 °C for 20 min. Wash 3 times with PBS.
(7) Add primary antibody: Anti-SAM monoclonal antibody was diluted at 1:400 with PBS containing 0.5% skimmed milk, 50 µL per well, 37°C wet-box for 1 h, and PBS washed 3 times.
(8) Add secondary antibody: FITC-labeled goat anti-mouse IgG diluted at 1:500 with PBS containing 0.5% non-fat dry milk, 50 µL per well, 37°C wet-box for 45 min, PBS washed 5 times (to avoid light); observed by ordinary fluorescence microscope and took pictures.
(9) 100 µL of 0.5 mg/ml DAPI (diamidino-2-phenylindole) was added and stained the cell nucleus for 20 min; after washing with PBS for 5 times, observed the cells under laser scan confocal microscope directly and photographed as needed.

Figures 5A through 5D show the comparison of the results of immunofluorescence staining of L02 and HepG2 cells after stimulation with 0.5 mM Met for 24 h and without Met under similar conditions as described above. Qualitative results indicated that SAM synthesis increased in L02 cells after 0.5 mM Met stimulation for 24 h, which was due to the increase of MAT-III/I activity; whereas the amount of SAM synthesis in Met treated HepG2 decreased since Met inhibited MAT-II activity. The results of immunofluorescence staining by laser scan confocal microscopy further confirmed the results above.

### EXAMPLE 6

### Determination of MAT activity purified from mouse liver

### Laboratory equipment:

PPS protein purification system (Institute of Engineering, Chinese Academy of Sciences); Ultrasonic crusher (Ningbo Xinzhi); High-speed refrigerated centrifuge (Xiangli Centrifuge)

### Reagent preparation:

Tissue homogenates: sucrose 0.25 M, Tris 10 mM, EGTA 0.1 M, beta-mercaptoethanol 0.1%. pH 7.5; Buffer A: MgSO₄ 10 mM, EDTA 1 mM, Tris 10 mM, pH 7.5; Buffer B: Buffer A + 600 mM KCl; Dialysate: Buffer A + 75 mM KCl; pH7.42 Enzyme Reaction Buffer: 100 mM Tris, 100 mM KCl, 20 mM MgSO₄, ProClin 1% adjusted to pH 7.42; 50% DMSO solution: Buffer A + equal volume of DMSO.

### Liver tissue homogenate:

1. Dissect mouse, remove connective tissue from liver, place it in a de-contracted 1.5 ml tube, and weigh the liver. The net weight was 0.6 g.
2. Wash the liver 3 to 5 times with 1 ml normal saline until the solution is clear and transparent.
3. The liver was cut into pieces and placed into a clean tissue homogenizer. About 3-fold more ice-cold tissue homogenate solution was added to the liver tissue pieces. Rotated and grinded in an ice bath for 6-8 min and grinded it thoroughly. The homogenate was removed, and the homogenizer was filled with 1 volume of hepatic tissue homogenate to wash the wall and aspirated into the homogenate.
4. The homogenate was placed in the liquid ice bath in an ultrasonic crusher for comminution, with ϕ6 probe, power 80%, working time was 3 seconds, pause time was 3 seconds, sonication was 30 minutes.
5. Homogenate was centrifuged under refrigerated condition at 15,000 x g high speed for 20 min, the supernatant was filtered with qualitative filter paper. A total of 150ml homogenate was obtained.
6. Slowly added 31.3g of ammonium sulfate per 100ml of the homogenate with stir, and added 47g of ammonium sulfate to make the saturation to be 50%. Stirred for 30 min and let it stand for 60 min.
7. Supernatant from high-speed refrigerated centrifuge was discarded. The precipitate was dissolved with 150 ml of pre-cooled dialysate, dialyzed in 2 L dialysate, and exchanged once.

### Purify with DEAE column:

1. Dialysis sample processing: The dialysis sample was taken out and centrifuged at high speed to discard precipitate. The supernatant is filtered with filter paper. About 230 ml of sample was obtained.
2. Took 60ml of DEAE packing, packed the column (3 x 20cm), and applied to the PPS chromatography system, rinsed with deionized water at a flow rate of 10ml/min for 30min, and equilibrated with dialysis buffer.
3. Mixed the sample and DEAE packing in a 4°C refrigerator using a low speed shaker for 90 minutes.
4. Packed the column, rinsed on the chromatographic system, and dialysis buffer was set to 6 ml/min for 30 min, set UV to zero.
5. Elution: 100% buffer B salt eluted for 20 min with a linear gradient, flow-rate was 6 ml/min. When the UV monitoring peak appeared, started to collect 5 ml per tube till the end of the peak, A total of 38 tubes were collected.

### MAT activity test for DEAE Eluent:

Used the PLL-SAM antigen coated ELSIA plate. Prepared MAT reaction system with reaction buffer with pH 7.42, ATP and Met at 2mM, and elution sample accounted for 50% of the reaction mixture. Control group had not ATP and Met substrates. The SAM quantification kit was used to measure the amount of SAM produced by each eluent, which indirectly reflected MAT activity and elution range. The activity unit was defined as the amount of SAM in nM produced per minute per milligram of MAT enzyme.

**Table 9 Reaction rates A/A0 of different DEAE eluent**

| | | | | | | | | | | |
|---|---|---|---|---|---|---|---|---|---|---|
| Elution 1 | 0.994 | Elution 9 | 0.938 | Elution 17 | 0.774 | Elution 25 | 0.985 | Elution 33 | 0.966 | MAT |
| Elution 2 | 0.981 | Elution 1 0 | 0.897 | Elution 18 | 0.782 | Elution 26 | 0.956 | Elution 34 | 1.004 | MAT |
| Elution 3 | 0.958 | Elution 11 | 0.880 | Elution 19 | 0.830 | Elution 27 | 0.950 | Elution 35 | 0.986 | ATP +Met |
| Elution 4 | 0.954 | Elution 12 | 0.829 | Elution 20 | 0.843 | Elution 28 | 0.975 | Elution 36 | 0.951 | Buffer |
| Elution 5 | 0.976 | Elution 13 | 0.864 | Elution 21 | 0.844 | Elution 29 | 0.966 | Elution 37 | 0.963 | MAT Control |
| Elution 6 | 0.985 | Elution 14 | 0.799 | Elution 22 | 0.865 | Elution 30 | 0.968 | Elution 38 | 1.002 | MAT Control |
| Elution 7 | 0.971 | Elution 15 | 0.732 | Elution 23 | 0.878 | Elution 31 | 0.995 | Pre-elution | 0.956 | Blank |
| Elution 8 | 0.961 | Elution 16 | 0.688 | Elution 24 | 0.949 | Elution 32 | 1.007 | FT | 0.943 | Blank |

The elution portion with the reaction rate A/A0 < 90% in Table 9 was significantly competing. The eluent from tubes 10-23 were pooled and dialyzed overnight to prepare for applying to a Phenyl Sepharose Fast Flow column. The protein concentration after dialysis was 5.98 mg/ml in a total volume of 70 ml. Activity of this component: 28 nM/60/0.015 ml x 5.95 mg/ml = 5.3 U (nM/min/mg).

### Phenyl Sepharose Fast Flow:

1. Took 20 ml of Phenyl Sepharose Fast Flow packing, packed a column, rinse with deionized water at a flow rate of 10 ml/min for 10 min, and equilibrated with buffer A + 220 mM KCl.
2. Mixed the sample and the packing in a 4°C refrigerator using a low speed shaker for 90 minutes.
3. Rinsed on the chromatographic system, set UV to zero.
4. Eluted with pre-cooled 50% DMSO solution at 6 ml/min. Collected elution peaks and dialyzed the eluent.

### Elution sample detection:

Three tubes of the eluent samples were pooled and assayed for MAT activity in the same manner as above with a load of 15 µl. SAM production was tested as 15 nM. The specific activity of this fraction was 15 nM/60/0.015 ml x 2.93 mg/ml = 5.7 U (nM/min/mg).

Due to the limited amount of sample left, no further purification was done. However, MAT activity has been measured after the purification with two chromatography columns. It is suggested that the method of determining MAT activity of the present invention is also applicable to the activity assay of MAT enzyme purified from mouse or rat liver.

### EXAMPLE 7

### Effect of different procedures on SAM synthesis

To clarify the effect of different steps or methods on the amount of SAM production, the MAT enzyme and the substrate were first applied to the PLL-aza-SAM coated strips using the optimal buffer and substrate concentrations described above. The reaction was carried out at 37°C for 20 min and reacted with HRP-anti-SAM antibody for 40 min. At the same time, MAT was directly reacted with the substrate and HRP-anti-SAM antibody for 1 h. The results showed that the amount of SAM synthesized by the step-by-step or two-step reaction was higher than that produced by the one-step method because the total volume of the system was only 30 µl during the first 20 min reaction. The concentrations of MAT and the substrates were both 2.33-fold higher than those of the one-step method. Table 10 shows the compared effects of these two different operating procedures on the amount of SAM synthesized. When the MAT concentration was lower at 0.3mg/ml, the SAM synthesized by the two-step method was 2.81 times higher than that of the one-step method. When the MAT concentration was 0.6mg/ml, the SAM synthesized by the two-step method was 1.76 times higher than that of the one-step method. When the MAT concentration was increased to 1 mg/ml, the SAM synthesized by the two-step method was 1.42 times higher than that of the one-step method. If MAT concentration continues to increase, it can be foreseen that the stepwise or two-step approach and one-step approach will be similar to each other in the SAM synthesis. The increases in the amount of SAM production (2.81, 1.76, 1.42 times) were not higher than the increases of the substrate and the MAT concentration (3.33 times). The stepwise method was helpful for the improvement of the SAM yield. Especially, in the test when MAT concentration or activity was low, the effects and advantages of the stepwise method would be more pronounced. If the MAT activity of the sample to be tested was high, and it was meant to compare the differences between groups of samples under different conditions, the one-step method is more convenient and easy, and the operating procedures have little influence on results.

**Table 10 Effects of different operating procedures on SAM synthesis**

| SAM (nM) | MAT Working Concentration (mg/ml) | | | | | |
|---|---|---|---|---|---|---|
| Methods | 0.3 | 0.3 | 0.6 | 0.6 | 1.0 | 1.0 |
| One-step | 189.0947 | 225.4812 | 352.0070 | 392.8603 | 521.8699 | 506.2779 |
| Two-step | 642.1810 | 520.4320 | 628.2861 | 681.0924 | 745.7204 | 713.5748 |
| Avg. fold increased | 2.81 | | 1.76 | | 1.42 | |

Numerous examples from the present invention demonstrate that the system we have established is very sensitive and can accurately and reliably determine the biological activity of MAT. Although the step-by-step method was used in this example, it was only used to selectively allow the MAT-catalyzed chemical reaction to proceed more efficiently in an independently optimal environment first, and immediately followed by performing quantitative immunological assay. In the second step 40-minute immunoassay, the MAT catalyzed chemical reaction continues to progress. The newly synthesized SAM immediately participates in the competitive immunological reaction. With the optimal system, all synthesized SAM molecules participate in the competition of tracer-labeled anti-SAM antibody. Due to the high specificity of the anti-SAM antibody, the results are very accurate There is no issue of inaccurate measurement caused by SAM loss due to various reasons. Therefore, the present invention proposes that both operating procedures are all very useful and can be used properly based on specific circumstances.

### EXAMPLE 8

### Determination of MAT activity using different coated antigens and standards from above

In addition to 1 µg/ml BSA-SAMe that was used for coating the ELISA plate, the same standard SAMe as the coating antigen was used as the standard antigen. HRP-anti-SAM 84-3 (Arthus Biosystems, MAH00202) was used as the anti-SAM antibody. Table 11 shows the result of such an experiment. The linearity of SAMe as a standard product was very good. When MAT was at 0.3 mg/ml, the SAM synthesized was about 3 µM. The result indicated that the use of SAM analogue aza-SAM plates and standard analogue as well as the SAMe plates and standard can both nicely reflect the changes of MAT activity. Due to differences in binding affinity or other factors, for the same densitometric readings, different amounts of antigens might be required or presented, resulting in differences in the values measured and the linear ranges of the two systems. However, the standard curves are linear and assays are sensitive enough to reflect the changes of MAT activity in both systems described in the present invention. Therefore, both of them may be used.

Several tests have proved that the results of using the SAMe coated plated and the standard in the present example were generally associated with poor repeatability and unstable linear range compared to those used in Examples 1-7 (coating the SAM analog antigen and using a stable analog as a standard). However, if the source of the raw material SAMe are well controlled and improved, and some related processes and methods are further improved and optimized, the solution in this example is also feasible.

**Table 11 Standard curve and results of SAMe for determining MAT activity**

| SAMe (uM) | OD450 (SAMe) | | OD450 (MAT) | MAT (mg/ml) |
|---|---|---|---|---|
| 0 | 1.0328 | 0.9483 | 0.3940 | 0.3 |
| 0.3125 | 1.0548 | 0.9196 | 0.4237 | 0.3 |
| 0.625 | 0.9742 | 0.9250 | 0.0448 | blank |
| 1.25 | 0.7431 | 0.7396 | 0.0473 | |
| 2.5 | 0.4976 | 0.4788 | 0.0450 | |
| 5 | 0.2461 | 0.2307 | 0.0473 | |
| 10 | 0.1389 | 0.1343 | 0.0453 | |
| 20 | 0.1407 | 0.1447 | 0.0460 | |

The above embodiment is a preferred embodiment of the present invention, but the embodiment of the present invention is not limited by the foregoing embodiment, and any other changes, modifications, substitutions, combinations, and other modifications made without departing from the spirit and principle of the present invention which are also possible and included in the present invention. Simplifications should all be equivalent to the replacement methods, and all are included in the protection scope of the claims of the present invention.

### References

1. Mato, J. M., Alvarez, L., Ortiz, P. & Pajares, M. A. S-adenosylmethionine synthesis: molecular mechanisms and clinical implications. Pharmacol.Ther. 1997; 73:265-280.
2. Lu SC, Mato JM. Role of methionine adenosyltransferase and SAMe in alcohol-associated liver cancer. Alcohol 2005; 35:227-234.
3. Kotb, M., Mudd, S. H., Mato, J. M., et al. Consensus nomenclature for the mammalian methionine adenosyltransferase genes and gene products. Trends Genet. 1997; 13:51-52.
4. Mudd, S. H. Activation of methionine for transmethylation. Enzyme-bound tripolyphosphate as an intermediate in the reaction catalyzed by the methionine-activating enzyme of bakers' yeast. J. Biol. Chem. 1963; 238:2156-2163.
5. Manuel M. Sa'nchez del Pino‡, Fernando J. et al. Hysteretic Behavior of Methionine Adenosyltransferase III - METHIONINE SWITCHES BETWEEN TWO CONFORMATIONS OF THE ENZYME WITH DIFFERENT SPECIFIC ACTIVITY. J. Biochem 2000; 275:23476-23482
6. Katz JE, Dlakic M, Clarke S. Automated identification of putative methyltransferases from genomic open reading frames. Mol. Cell. Proteomics 2003; 2:525-540.
7. Fernandez-Irigoyen J, Santamaría E, Chien YH, et al. Enzymatic activity of methionine adenosyltransferase variants identified in patients with persistent hypermethioninemia. Molecular Genetics and Metabolism. 2010; 101:172-177.
8. Mato JM, Corrales FJ, Lu SC, et al. S-Adenosylmethionine: a control switch that regulates liver function, FASEB J. 2002; 16:15-26.
9. Avila MA, Berasain C, Torres L, et al. Reduced mRNA abundance of the main enzymes involved in methionine metabolism in human liver cirrhosis and hepatocellular carcinoma, J. Hepatol. 2000; 33:907-914.
10. Lu SC, Alvarez L, Huang ZZ, et al. Methionine adenosyltransferase 1A knockout mice are predisposed to liver injury and exhibit increased expression of genes involved in proliferation, Proc. Natl Acad. Sci. USA 2001; 98:5560-5565.
11. Martinez-Chantar ML, Vázquez-Chantada M, Ariz U, et al. Loss of glycine Nmethyltransferase gene leads to steatosis and hepatocellular carcinoma in mice. Hepatology. 2008; 47:1191-1199.
12. Martinez-Chantar ML, Latasa MU, Varela-Rey M, et al. L-Methionine Availability Regulates Expression of the Methionine Adenosyltransferase 2A Gene in Human Hepatocarcinoma Cells. J Biochem. 2003; 278(22):19885-19890.

## Claims

1. The use of anti-S-adenosylmethionine-specific antibodies for determining the biological activity of methionine adenosyltransferase.

2. A method for determining the biological activity of methionine adenosyltransferase in a sample, said method comprising the following steps:
(a) reacting in a buffer system that preserves the biological activity of methionine adenosyltransferase, the sample containing said enzyme and substrates to form S-adenosylmethionine; and
(b) using an immunological method to measure the concentration of S-adenosylmethionine to determine whether methionine adenosyltransferase activity is present in said reaction system and determine the level of said methionine adenosyltransferase activity.

3. The method according to claim 2, wherein the substrate contains a buffer system of methionine, adenosine triphosphate, magnesium ions and potassium ions with suitable acidity and alkalinity.

4. The method according to claim 2, wherein the sample is derived from the group consisting of: genetic engineering products, purified samples from biological tissue cells, methionine adenosyltransferases in tissue cells, biological fluids or tissue and cell cultured fluids; biological fluids including blood, plasma, serum, saliva, urine, cerebrospinal fluid, abdominal or thoracic exudates and tissue fluids.

5. The method according to claim 2, wherein said immunological method includes using of one of the following components: (a) a medium coated with S-adenosylmethionine or its analogues, or a medium coated with an anti-S-adenosylmethionine specific antibody, (b) correspondingly, an tracer-labeled anti-S-adenosylmethionine specific antibody, or an tracer-labeled S-adenosylmethionine or its analogues, and (c) a corresponding tracer detection system.

6. The method according to claim 2, wherein the catalytic reaction of methionine adenosyltransferase and immunoassay to measure the resulting product S-adenosylmethionine that competes with the coated S-adenosylmethionine antigen for the binding of tracer-labeled anti-S-adenosylmethionine antibodies, or competes with tracer-labeled S-adenosylmethionine antigens for the binding of coated anti-S-adenosylmethionine antibodies, can be simultaneous in one reaction system in one-step or two-step format.

7. An assay kit for determining the biological activity of methionine adenosyltransferase comprising the following components:
(a) a micro-titer plate coated with S-adenosyl-methionine antigen or its analogues, or anti-S-adenosylmethionine antibody;
(b) a tracer labeled anti-S-adenosylmethionine antibody, or tracer labeled S-adenosylmethionine antigen or labeled S-adenosylmethionine analog;
(c) an S-adenosylmethionine standard;
(d) an enzyme substrate comprising methionine, adenosine triphosphate in appropriate buffer;
(e) an MAT positive control;
(f) a tracer detection system; and
(g) a buffer with appropriate pH.

8. The assay kit according to claim 7, wherein the said micro-titer plate is coated with polylysine, bovine serum albumin or other carrier protein-conjugated S-adenosylmethionine or S-adenosylmethionine analogues, or it is coated directly with anti-S-adenosylmethionine antibodies (monoclonal antibodies or polyclonal antibodies) or indirectly with the S-adenosylmethionine monoclonal antibody through goat or rabbit anti-mouse IgG antibody.

9. A one-step method of claim 6 for determining the biological activity of methionine adenosyltransferase in a sample using the assay kit according to claim 7, said method comprising the following steps:
(a) add different concentrations of the standard solutions from said kit to the wells of a micro-titer plate from said kit coated with protein-SAM (or SAM analogues) conjugates or a polymer-SAM (or SAM analogues) conjugates;
(b) add samples to be tested and methionine adenosyltransferase positive control to other wells;
(c) add horseradish peroxidase or alkaline phosphatase conjugated anti-S-adenosylmethionine monoclonal antibody, methionine and adenosine triphosphate substrate buffer, mix and incubate at 37°C for 60 minutes and wash the plate after incubation;
(d) add horseradish peroxidase or alkaline phosphatase substrate, allow color development at 37 °C for 15 minutes followed by stop solution to terminate the reaction and then in the plate reader read optical absorbance at 450nm wavelength OD450 per well;
(e) plot the graph according to the known concentrations of the standard and the corresponding values of OD450, the curve equation can be obtained;
(f) the sample OD450 is then substituted into the equation to obtain the corresponding concentration of SAM produced; and
(g) calculate the methionine adenosyltransferase activity of the sample containing methionine adenosyltransferase based on the concentration of the synthesized SAM per unit time.

10. A two-step method of claim 6 for determining the biological activity of methionine adenosyltransferase in a sample using the assay kit according to claim 7, said method comprising the following steps:
(a) add samples to be tested and methionine adenosyltransferase positive control to the methionine and adenosine triphosphate substrate buffer in separate tubes and incubate at 37°C for 20 minutes;
(b) add the mixtures from step (a) and different concentrations of said standard solutions to the wells of a micro-titer plate from said kit coated with protein-SAM (or SAM analogues) conjugates or a polymer-SAM (or SAM analogues) conjugates;
(c) add horseradish peroxidase or alkaline phosphatase conjugated anti-S-adenosylmethionine monoclonal antibody, mix and incubate at 37°C for 40 minutes and wash the plate after incubation;
(d) add horseradish peroxidase or alkaline phosphatase substrate, allow color development at 37 °C for 15 minutes followed by stop solution to terminate the reaction and then in the plate reader read optical absorbance at 450nm wavelength OD450 per well;
(e) plot the graph according to the known concentrations of the standard and the corresponding values of OD450, the curve equation can be obtained;
(f) the sample OD450 is then substituted into the equation to obtain the corresponding concentration of SAM produced; and
(g) calculate the methionine adenosyltransferase activity of the sample containing methionine adenosyltransferase based on the concentration of the synthesized SAM per unit time.

11. The method according to claim 5 and 6 or assay kit according to claim 7, wherein the tracer is selected from the group consisting of enzymes, fluorescein, colloidal gold, chemiluminescent substances, biotin, digoxin (or digoxigenin), radiolabeled substances and various types of latex microspheres.

12. The assay kit according to claim7, wherein the buffer system comprises a magnesium ion concentration in the range of about 20-100 mM, a potassium ion concentration in the range of about 50-400 mM, a tris (hydroxymethyl) aminomethane hydrochloric acid concentration in the range of about 50-200 mM, and a pH range of about 7.42-8.5.
